# EUROPEAN PATENT SPECIFICATION

(11) **EP 4 312 993 B1**
(45) Date of publication and mention of the grant of the patent: **08.10.2025**
(21) Application number: 22717111.3
(22) Date of filing: 21.03.2022
(51) Int. Cl.: A61K 9/20, A61K 31/5377, A61P 35/00

(54) **FORMULATION COMPRISING CERALASERTIB**
FORMULIERUNG MIT CERALASERTIB
FORMULATION COMPRENANT DU CÉRALASERTIB

(30) Priority: 22.03.2021 WO PCT/CN2021/081978
(43) Date of publication of application: 07.02.2024
(73) Proprietor: Astrazeneca AB, 151 85 Södertälje (SE)
(72) Inventor: SIMPSON, David Bradley Brook, Cambridge Cambridgeshire CB2 0AA (GB); REN, Haixia, Shanghai (CN)
(74) Representative: AstraZeneca Intellectual Property
(86) International application number: PCT/EP2022/057305
(87) International publication number: WO 2022/200251

(56) References cited:
- WO-A1-2018/153972
- CN-A- 112 043 831
- DILLON M.T. ET AL: "PATRIOT: A phase I study to assess the tolerability, safety and biological effects of a specific ataxia telangiectasia and Rad3-related (ATR) inhibitor (AZD6738) as a single agent and in combination with palliative radiation therapy in patients with solid tumours", CLINICAL AND TRANSLATIONAL RADIATION ONCOLOGY, vol. 12, 8 June 2018 (2018-06-08), pages 16 - 20, XP055940488, ISSN: 2405-6308, Retrieved from the Internet <URL:https://www.ncbi.nlm.nih.gov/pmc/articles/PMC6068075/pdf/main.pdf> DOI: 10.1016/j.ctro.2018.06.001

## Description

### FIELD

The present specification relates to a pharmaceutical formulation comprising ceralasertib, more specifically, a pharmaceutical formulation which comprises ceralasertib, dibasic calcium phosphate, microcrystalline cellulose, low-substituted hydroxypropyl cellulose and magnesium stearate. The specification also relates to the formulation for use in therapy, particularly for use in the treatment of cancer.

### BACKGROUND

Ataxia Telangiectasia and Rad3 related protein (ATR) is a serine/threonine protein kinase and member of the phosphatidylinositol 3-kinase related kinase (PIKK) family. During normal DNA replication, ATR is recruited at stalled replication forks, which can progress to double strand breaks if left unrepaired. ATR is also recruited to single strand DNA coated with Replication Protein A (RPA) following single strand DNA damage or the resection of double strand breaks. Recruitment and activation of ATR leads to cell cycle arrest in the S-phase while the DNA is repaired and the stalled replication fork resolved, or nuclear fragmentation and entry into programmed cell death (apoptosis).

As a result, ATR inhibitors are expected to cause growth inhibition in tumour cells dependent upon ATR for DNA repair e.g. ataxia telangiectasia mutated (ATM) deficient tumours. In addition to such monotherapy activity, ATR inhibitors are also predicted to potentiate the activity of cytotoxic DNA damaging agents and radiotherapy (through inhibition of ATR-dependent DNA repair processes) when used in combination.

Ceralasertib is a potent inhibitor of ATR with good selectivity against other PIKK family members, and was first disclosed in WO2011/154737. It is being developed as an oral anti-tumour agent in patients with disease that is dependent upon ATR function for DNA repair, for example tumours that are deficient of ATM. Ceralasertib is being specifically evaluated for the treatment of breast cancer, for example triple negative breast cancer; gastric cancer; chronic lymphocytic leukaemia (CLL); head and neck squamous cell carcinoma (H&NSCC); non-small cell lung cancer (NSCLC); ovarian cancer; non-Hodgkin's lymphoma (NHL); small cell lung cancer (SCLC); and melanoma.

DILLON M.T. ET AL: "PATRIOT: A phase I study to assess the tolerability, safety and biological effects of a specific ataxia telangiectasia and Rad3-related (ATR) inhibitor (AZD6738) as a single agent and in combination with palliative radiation therapy in patients with solid tumours", CLINICAL AND TRANSLATIONAL RADIATION ONCOLOGY, vol. 12, 8 June 2018 (2018-06-08), pages 16-20, discloses a phase I study of the ATR inhibitor AZD6738 as monotherapy and in combination with palliative radiotherapy.

Patient compliance, even with life threatening conditions like cancer, is a serious threat to treatment. A study conducted for the US company CVS Pharmacy reported that the average adherence rate (the degree to which patients correctly follow prescription instructions) for medicines taken only once daily is nearly 80%, compared to about 50 % for treatments that must be taken 4 times a day. As many as 75% of patients (and 50% of chronically ill patients) fail to adhere to, or comply with, physician prescribed treatment regimens. Current dosages of ceralasertib under evaluation are 160 mg qd and 240mg bd. This requires a formulation with high drug loading in order to mitigate patient compliance risk caused by taking multiple tablets. A higher drug loading can also contribute to a lower overall cost of goods.

In addition to drug loading, a suitable ceralasertib formulation must also satisfy several other criteria to be suitable for commercial scale production. Ceralasertib exhibits poor flowability and is a slightly hygroscopic crystalline material which limits some of the manufacturing techniques that may be employed. A suitable formulation must have good manufacturability (the extent to which a product can be manufactured with relative ease at minimum cost and maximum reliability) because a robust process is needed to deliver product for late-stage clinical trials and commercial purposes. Where the formulation is a tablet, the tablets must achieve a suitable tensile strength, without the need for the application of a high compression force, whilst maintaining an acceptable tablet porosity. These factors minimise the risk of over-compression and variable dissolution rates - a suitable formulation must release its active ingredient in a uniform and acceptable manner. Finally, a formulation for human use must employ pharmaceutically acceptable excipients that meet regulatory requirements for chronic dosing, and must be stable for a meaningful period of time, typically > 1 year and ideally up to 3 years.

The present specification describes a pharmaceutical formulation of ceralasertib that satisfies these criteria and is suitable for manufacture on a commercial scale.

### SUMMARY

The invention is defined by the claims. Any subject-matter falling outside the scope of the claims is provided for information purposes only.

This specification describes, in part, a pharmaceutical formulation which comprises ceralasertib, dibasic calcium phosphate, microcrystalline cellulose, low-substituted hydroxypropyl cellulose and magnesium stearate.

This specification also describes, in part, the above-mentioned pharmaceutical formulation for use as a medicament.

This specification also describes, in part, the above-mentioned pharmaceutical formulation for use in a method of treating cancer in a warm-blooded animal, such as a man, which comprises administering to said animal an effective amount of said pharmaceutical formulation.

This specification also describes, in part, the use of a pharmaceutical formulation in the manufacture of a medicament for the treatment of cancer in a warm-blooded animal such as a man. This is a not claimed embodiment.

This specification also describes, in part, the above-mentioned pharmaceutical formulation for use in the treatment of cancer.

### DETAILED DESCRIPTION

Many embodiments are detailed throughout the specification and will be apparent to a reader skilled in the art. The specification is not to be interpreted as being limited to any of the recited embodiments.

"A" means "at least one". In any embodiment where "a" is used to denote a given material or element, "a" may mean one.

"Comprising" means that a given material or element may contain other materials or elements. In any embodiment where "comprising" is mentioned the given material or element may be formed of at least 10% w/w, at least 20% w/w, at least 30% w/w, or at least 40% w/w of the material or element. In any embodiment where "comprising" is mentioned, "comprising" may also mean "consisting of" (or "consists of") or "consisting essentially of" (or "consists essentially of") a given material or element.

"Consisting of" or "consists of" means that a given material or element is formed entirely of the material or element. In any embodiment where "consisting of" or "consists of" is mentioned the given material or element may be formed of 100% w/w of the material or element.

"Consisting essentially of" or "consists essentially of" means that a given material or element consists almost entirely of that material or element. In any embodiment where "consisting essentially of" or "consists essentially of" is mentioned the given material or element may be formed of at least 50% w/w, at least 60% w/w, at least 70% w/w, at least 80% w/w, at least 90% w/w, at least 95% w/w or at least 99% w/w of the material or element.

"w/w" means weight by weight.

In any embodiment where "is" or "may be" is used to define a material or element, "is" or "may be" may mean the material or element "consists of" or "consists essentially of" the material or element.

As mentioned above, the invention is defined by the claims.

### Ceralasertib

Ceralasertib, also known as AZD6738, is 4-{4-[(3R)-3-methylmorpholin-4-yl]-6-[1-((R)-S-methylsulfonimidoyl)cyclopropyl]pyrimidin-2-yl}-1H-pyrrolo[2,3-b]pyridine, has the chemical structure:

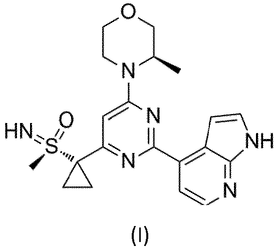

In one embodiment, ceralasertib refers to free base ceralasertib.

In one embodiment, ceralasertib refers to ceralasertib and pharmaceutically-acceptable salts thereof.

In one embodiment, ceralasertib refers to a pharmaceutically-acceptable salt of ceralasertib.

In one embodiment, the pharmaceutical formulation described herein contains >25 % w/w ceralasertib.

In one embodiment, the pharmaceutical formulation described herein contains 30-50 % w/w ceralasertib.

In one embodiment, the pharmaceutical formulation described herein contains 35-45 % w/w ceralasertib.

In one embodiment, the pharmaceutical formulation described herein contains 37.5-42.5 % w/w ceralasertib.

In one embodiment, the pharmaceutical formulation described herein contains about 40% w/w ceralasertib.

In one embodiment, the pharmaceutical formulation described herein contains 40% w/w ceralasertib.

In one embodiment, ceralasertib refers to ceralasertib with particle size ≤500µm.

In one embodiment, ceralasertib refers to ceralasertib with particle size ≤150µm.

In one embodiment, ceralasertib refers to ceralasertib with particle size ≤90µm.

Ceralasertib may exist in one of two crystalline forms - ceralasertib Form A and ceralasertib Form B.

### Form A

Ceralasertib Form A has an X-ray powder diffraction (XRPD) pattern with at least one of the following 2-theta (2θ) values measured using CuKα radiation: 21.2 and 17.2°. The ten most prominent X-Ray powder diffraction peaks are shown in Table 1:

**Table 1**

| **Angle 2-Theta (2θ)** | **Intensity** |
|---|---|
| 21.2 | 5276 |
| 17.2 | 3615 |
| 18.7 | 3232 |
| 12.5 | 3184 |
| 11.0 | 2823 |
| 17.0 | 2768 |
| 23.7 | 2705 |
| 22.8 | 2507 |
| 10.0 | 2190 |
| 14.4 | 1931 |

Ten X-Ray Powder Diffraction peaks for ceralasertib Form A wherein the 2-theta values are +/-0.2°

In one embodiment, ceralasertib refers to ceralasertib Form A.

In one embodiment, ceralasertib refers to ceralasertib Form A with an X-ray powder diffraction pattern with at least one specific peak at about 2-theta = 21.2°.

In one embodiment, ceralasertib refers to ceralasertib Form A with an X-ray powder diffraction pattern with at least one specific peak at about 2-theta = 17.2°.

In one embodiment, ceralasertib refers to ceralasertib Form A with an X-ray powder diffraction pattern with at least two specific peaks at about 2-theta = 21.2° and 17.2°.

In one embodiment, ceralasertib refers to ceralasertib Form A with an X-ray powder diffraction pattern with specific peaks at about 2-theta = 21.2, 17.2, 18.7, 12.5, 11.0, 17.0, 23.7, 22.8, 10.0, 14.4°.

In one embodiment, the 2-theta values may be plus or minus 0.2° theta.

DSC analysis of ceralasertib Form A shows a melting endotherm with an onset between 221.5°C and 222.1°C, for example 221.8°C and a peak at between 222.0°C and 222.6°C, for example 222.3°C.

In one embodiment, ceralasertib refers to ceralasertib Form A with an onset of melting at about 221.8°C and a peak at about 222.3°C.

### Form B

Ceralasertib Form B has an X-ray powder diffraction pattern with at least one of the following 2θ values measured using CuKα radiation: 13.2 and 18.3°. The ten most prominent X-Ray powder diffraction peaks are shown in Table 2:

**Table 2**

| **Angle 2-Theta (2θ)** | **Intensity** |
|---|---|
| 13.2 | 10183 |
| 18.3 | 5715 |
| 19.7 | 5711 |
| 21.7 | 5469 |
| 15.9 | 5226 |
| 13.0 | 5248 |
| 28.1 | 5153 |
| 9.6 | 4795 |
| 14.2 | 4587 |
| 10.1 | 4267 |

Ten X-Ray Powder Diffraction peaks for ceralasertib Form B wherein the 2-theta values are +/- 0.2°

In one embodiment, ceralasertib refers to ceralasertib Form B.

In one embodiment, ceralasertib refers to ceralasertib Form B with an X-ray powder diffraction pattern with at least one specific peak at about 2-theta = 13.2°.

In one embodiment, ceralasertib refers to ceralasertib Form B with an X-ray powder diffraction pattern with at least one specific peak at about 2-theta = 18.3°.

In one embodiment, ceralasertib refers to ceralasertib Form B with an X-ray powder diffraction pattern with at least two specific peaks at about 2-theta = 13.2° and 18.3°.

In one embodiment, ceralasertib refers to ceralasertib Form B with an X-ray powder diffraction pattern with specific peaks at about 2-theta = 13.2, 18.3, 19.7, 21.7, 15.9, 13.0, 28.1, 9.6, 14.2, 10.1°.

In one embodiment, the 2-theta values may be plus or minus 0.2° theta.

DSC analysis of ceralasertib Form B shows a melting endotherm with an onset between 52.7°C and 53.3°C, for example 53.0°C and a peak at between 53.9°C and 54.5°C, for example 54.2°C.

In one embodiment, ceralasertib refers to ceralasertib Form B with an onset of melting at about 53.0°C and a peak at about 54.2°C.

When it is stated that the present specification relates to a crystalline form of ceralasertib, i.e. Form A or Form B, in one embodiment the degree of crystallinity is greater than about 60%, greater than about 80%, greater than about 90%, or greater than about 95%. In one embodiment the degree of crystallinity is greater than about 98%.

### Dibasic calcium phosphate

Dibasic calcium phosphate is the calcium phosphate with the formula CaHPO₄ and is used as a water insoluble filler. It improves the flow of ceralasertib due to its high bulk density. Dibasic calcium phosphate is referred to as calcium hydrogen phosphate in the European Pharmacopoeia, and anhydrous dibasic calcium phosphate in the US and Japanese Pharmacopoeias.

Dibasic calcium phosphate exists in two main forms - the dihydrate and the anhydrous form.

In one embodiment dibasic calcium phosphate refers to dibasic calcium phosphate dihydrate.

In one embodiment dibasic calcium phosphate refers to anhydrous dibasic calcium phosphate.

In one embodiment, the pharmaceutical formulation described herein contains <16.5% w/w anhydrous dibasic calcium phosphate.

In one embodiment, the pharmaceutical formulation described herein contains 10-16% w/w anhydrous dibasic calcium phosphate.

In one embodiment, the pharmaceutical formulation described herein contains 12-16% w/w anhydrous dibasic calcium phosphate.

In one embodiment, the pharmaceutical formulation described herein contains about 15% w/w anhydrous dibasic calcium phosphate.

In one embodiment, the pharmaceutical formulation described herein contains 15% w/w anhydrous dibasic calcium phosphate.

In one embodiment dibasic calcium phosphate refers to calcium hydrogen phosphate of European Pharmacopoeial standard.

In one embodiment dibasic calcium phosphate refers to anhydrous dibasic calcium phosphate of Japanese Pharmacopoeial standard.

In one embodiment dibasic calcium phosphate refers to anhydrous dibasic calcium phosphate of United States Pharmacopeial standard.

### Microcrystalline cellulose

Microcrystalline cellulose or "cellulose, microcrystalline" is also used as a filler. Microcrystalline cellulose is non-digestible plant matter from sources like wood pulp and tough plant stalks like sorghum, cotton linen or hemp. The plants are harvested, cleaned and ground to create a fine, white powder whose tiny crystals can be viewed under a microscope. Microcrystalline cellulose is the same as cellulose, except that it meets pharmacopeia standards; and it is available in different grades and particle sizes. It may also be referred to as powdered cellulose, cellulose gum or carboxymethylcellulose.

In one embodiment microcrystalline cellulose refers to microcrystalline cellulose with a particle size of about 40-200µM.

In one embodiment microcrystalline cellulose refers to microcrystalline cellulose with a particle size of about 80-120µM.

In one embodiment microcrystalline cellulose refers to microcrystalline cellulose with a particle size of about 100µM.

In one embodiment microcrystalline cellulose refers to Avicel^{®} PH-102 microcrystalline cellulose.

In one embodiment, the pharmaceutical formulation described herein contains 20-50% w/w microcrystalline cellulose.

In one embodiment, the pharmaceutical formulation described herein contains 30-40% w/w microcrystalline cellulose.

In one embodiment, the pharmaceutical formulation described herein contains 30-36% w/w microcrystalline cellulose.

In one embodiment, the pharmaceutical formulation described herein contains 33-34% w/w microcrystalline cellulose.

In one embodiment, the pharmaceutical formulation described herein contains about 33.5% w/w microcrystalline cellulose.

In one embodiment, the pharmaceutical formulation described herein contains 33.5% w/w microcrystalline cellulose.

In one embodiment microcrystalline cellulose refers to cellulose, microcrystalline of European Pharmacopoeial standard.

In one embodiment microcrystalline cellulose refers to microcrystalline cellulose of Japanese Pharmacopoeial standard.

In one embodiment microcrystalline cellulose refers to microcrystalline cellulose of United States Pharmacopeial standard.

### Low-substituted hydroxypropyl cellulose

Low substituted hydroxypropyl cellulose (L-HPC), also known as "hydroxypropyl cellulose, low-substituted", is used as a disintegrant. Disintegrants allow for breakdown when wet ensuring rapid breakdown to facilitate rapid absorption of a product. L-HPC is insoluble in water, and it is an effective disintegrant due to its swelling action in water.

In one embodiment, the pharmaceutical formulation described herein contains 5-20% w/w low substituted hydroxypropyl cellulose.

In one embodiment, the pharmaceutical formulation described herein contains 7.5-12.5% w/w low substituted hydroxypropyl cellulose.

In one embodiment, the pharmaceutical formulation described herein contains about 10% w/w low substituted hydroxypropyl cellulose.

In one embodiment, the pharmaceutical formulation described herein contains 10% w/w low substituted hydroxypropyl cellulose.

In one embodiment, the low substituted hydroxypropyl cellulose is LH-11 grade low substituted hydroxypropyl cellulose.

In one embodiment, the low substituted hydroxypropyl cellulose is LH-21 grade low substituted hydroxypropyl cellulose.

In one embodiment, the low substituted hydroxypropyl cellulose is LH-22 grade low substituted hydroxypropyl cellulose.

In one embodiment, the low substituted hydroxypropyl cellulose is LH-31 grade low substituted hydroxypropyl cellulose.

In one embodiment, the low substituted hydroxypropyl cellulose is LH-32 grade low substituted hydroxypropyl cellulose.

In one embodiment, the low substituted hydroxypropyl cellulose is LH-B1 grade low substituted hydroxypropyl cellulose.

In one embodiment low substituted hydroxypropyl cellulose refers to hydroxypropyl cellulose, low-substituted of European Pharmacopoeial standard.

In one embodiment low substituted hydroxypropyl cellulose refers to low-substituted hydroxypropyl cellulose of National Formulary standard.

In one embodiment low substituted hydroxypropyl cellulose refers to low substituted hydroxypropyl cellulose of Japanese Pharmacopoeial standard.

### Magnesium stearate

Magnesium stearate has the formula Mg(C₁₈H₃₅O₂)₂. It is used as a lubricant to reduce friction and is typically a white, water-insoluble powder.

In one embodiment, the pharmaceutical formulation described herein contains 0.5-2.5 % w/w magnesium stearate.

In one embodiment, the pharmaceutical formulation described herein contains 1-2 % w/w magnesium stearate.

In one embodiment, the pharmaceutical formulation described herein contains about 1.5% w/w magnesium stearate.

In one embodiment, the pharmaceutical formulation described herein contains 1.5% w/w magnesium stearate.

In one embodiment, the magnesium stearate is MF-2-V grade magnesium stearate.

In one embodiment magnesium stearate refers to magnesium stearate of United States Pharmacopoeial standard.

In one embodiment magnesium stearate refers to magnesium stearate of British Pharmacopoeial standard.

In one embodiment magnesium stearate refers to magnesium stearate of European Pharmacopoeial standard.

In one embodiment magnesium stearate refers to magnesium stearate of Japanese Pharmacopoeial standard.

In one embodiment magnesium stearate refers to magnesium stearate of Chinese Pharmacopoeial standard.

In one embodiment, during processing the magnesium stearate may be added in two batches: (i) before granulation step (intra); and (ii) in the final blending process (extra).

In one embodiment, about 1/3 of the magnesium stearate may be intra magnesium stearate; and about 2/3 of the magnesium stearate may be extra magnesium stearate.

### Pharmacopoeia

Herein where pharmacopeia standards are mentioned, it is to be understood that the standards are as published in the following editions of the pharmacopoeias:

**Table 3: Pharmacopeia Editions**

| **Pharmacopoeia** | **Edition** |
|---|---|
| United States Pharmacopoeia | 43 |
| British Pharmacopoeia | 2020 Edition |
| European Pharmacopoeia | 10^{th} Edition |
| Japanese Pharmacopoeia | 17^{th} Edition |
| Chinese Pharmacopoeia | 2020 Edition |
| National Formulary | 38 |

### Pharmaceutically-acceptable salts

As used herein, "pharmaceutically acceptable salts" refers to derivatives wherein the parent compound is modified by converting an existing acidic moiety (e.g. carboxyl and the like) or base moiety (e.g. amine, alkali and the like) to its salt form. Suitable pharmaceutically acceptable salts include, for example, an acid-addition salt, which can be derived from for example an inorganic acid (for example, hydrochloric, hydrobromic, sulfuric, phosphoric acid and the like) or organic acid (for example, maleic, fumaric, citric, methanesulfonic and the like).

Suitable pharmaceutically acceptable salts also include, for example, a base-addition salt, which can be derived from for example an inorganic base (for example, sodium or potassium, salts of metals from columns I to XII of the periodic table such as calcium, magnesium and the like) or organic bases (for example, primary, secondary, and tertiary amines, substituted amines including naturally occurring substituted amines, cyclic amines, basic ion exchange resins, and the like). Certain organic amines include but are not limited to triethylamine, ethanolamine, diethanolamine, triethanolamine, morpholine, N-methylpiperidine, N-ethylpiperidine, dibenzylamine or amino acids such as lysine. The skilled person would appreciate that adding acids or bases for forming acid/base-addition salts other than those shown in the examples may also be possible. In respect of salts, only the pharmaceutically acceptable salts of ceralasertib belong to the present invention. Lists of additional suitable salts can be found, e.g. in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

### Tablet

In one embodiment, the pharmaceutical formulation described herein may be a tablet.

In one embodiment, the pharmaceutical formulation described herein may be a tablet for oral administration.

In one embodiment, the pharmaceutical formulation described herein may be an immediate release tablet.

In one embodiment, the pharmaceutical formulation described herein may be administered daily.

In one embodiment, the pharmaceutical formulation described herein may be a tablet administered daily.

In one embodiment, the pharmaceutical formulation described herein may be administered twice a day (bd).

In one embodiment, the pharmaceutical formulation described herein may be a tablet administered twice a day (bd).

In one embodiment, the pharmaceutical formulation described herein may comprise 80 - 160mg of ceralasertib.

In one embodiment, the pharmaceutical formulation described herein may be a tablet comprising 80 - 160mg of ceralasertib.

In one embodiment, the pharmaceutical formulation described herein may comprise 100 - 140mg of ceralasertib.

In one embodiment, the pharmaceutical formulation described herein may be a tablet comprising 100 -140mg of ceralasertib.

In one embodiment, the pharmaceutical formulation described herein may comprise about 80mg of ceralasertib.

In one embodiment, the pharmaceutical formulation described herein may be a tablet comprising about 80mg of ceralasertib.

In one embodiment, the pharmaceutical formulation described herein may comprise 80mg of ceralasertib.

In one embodiment, the pharmaceutical formulation described herein may be a tablet comprising 80mg of ceralasertib.

In one embodiment, the pharmaceutical formulation described herein may comprise about 80mg of ceralasertib and be administered twice a day.

In one embodiment, the pharmaceutical formulation described herein may be a tablet comprising about 80mg of ceralasertib and be administered twice a day.

In one embodiment, the pharmaceutical formulation described herein may comprise 80mg of ceralasertib and be administered twice a day.

In one embodiment, the pharmaceutical formulation described herein may be a tablet comprising 80mg of ceralasertib and be administered twice a day.

In one embodiment, the pharmaceutical formulation described herein may comprise about 120mg of ceralasertib.

In one embodiment, the pharmaceutical formulation described herein may be a tablet comprising about 120mg of ceralasertib.

In one embodiment, the pharmaceutical formulation described herein may comprise 120mg of ceralasertib.

In one embodiment, the pharmaceutical formulation described herein may be a tablet comprising 120mg of ceralasertib.

In one embodiment, the pharmaceutical formulation described herein may comprise about 120mg of ceralasertib and be administered twice a day.

In one embodiment, the pharmaceutical formulation described herein may be a tablet comprising about 120mg of ceralasertib and be administered twice a day.

In one embodiment, the pharmaceutical formulation described herein may comprise 120mg of ceralasertib and be administered twice a day.

In one embodiment, the pharmaceutical formulation described herein may be a tablet comprising 120mg of ceralasertib and be administered twice a day.

In one embodiment, the pharmaceutical formulation described herein may comprise about 160mg of ceralasertib.

In one embodiment, the pharmaceutical formulation described herein may be a tablet comprising about 160mg of ceralasertib.

In one embodiment, the pharmaceutical formulation described herein may comprise 160mg of ceralasertib.

In one embodiment, the pharmaceutical formulation described herein may be a tablet comprising 160mg of ceralasertib.

In one embodiment, the pharmaceutical formulation described herein may comprise about 160mg of ceralasertib and be administered twice a day.

In one embodiment, the pharmaceutical formulation described herein may be a tablet comprising about 160mg of ceralasertib and be administered twice a day.

In one embodiment, the pharmaceutical formulation described herein may comprise 160mg of ceralasertib and be administered twice a day.

In one embodiment, the pharmaceutical formulation described herein may be a tablet comprising 160mg of ceralasertib and be administered twice a day.

In one embodiment, the pharmaceutical formulation described herein may comprise:
30-50% w/w ceralasertib;
10-16% w/w anhydrous dibasic calcium phosphate;
30-40% w/w microcrystalline cellulose;
5-20% w/w low substituted hydroxypropyl cellulose; and
0.5-2.5 % w/w magnesium stearate.

In one embodiment, the pharmaceutical formulation described herein may comprise:
40% w/w ceralasertib;
15% w/w anhydrous dibasic calcium phosphate;
33.5% w/w microcrystalline cellulose;
10% w/w low substituted hydroxypropyl cellulose; and
1.5% w/w magnesium stearate.

In one embodiment, the pharmaceutical formulation described herein may be a tablet comprising:
30-50 % w/w ceralasertib;
10-16% w/w anhydrous dibasic calcium phosphate;
30-40% w/w microcrystalline cellulose;
5-20% w/w low substituted hydroxypropyl cellulose; and
0.5-2.5 % w/w magnesium stearate.

In one embodiment, the pharmaceutical formulation described herein may be a tablet comprising:
40% w/w ceralasertib;
15% w/w anhydrous dibasic calcium phosphate;
33.5% w/w microcrystalline cellulose;
10% w/w low substituted hydroxypropyl cellulose; and
1.5% w/w magnesium stearate.

### Coating

In one embodiment, where the pharmaceutical formulation is in the form of a tablet, it may be advantageous to coat the tablet with a coating. The resultant coated tablet would then comprise a tablet core and a coating. A coated tablet may assist a patient in swallowing a tablet. A coloured coating may assist with patient compliance and/or differentiation from other medicines, particularly those administered as part of a combination treatment. A pink tablet may be particularly suitability in terms of differentiation.

In one embodiment, the pharmaceutical formulation described herein may be a coated tablet. If the tablet is a coated tablet it is to be understood that any % w/w value quoted herein refers to the w/w composition of the tablet core.

In one embodiment, the pharmaceutical formulation described herein may be a colour coated tablet.

In one embodiment, the pharmaceutical formulation described herein may be a film-coated tablet.

In one embodiment, the pharmaceutical formulation described herein may be a coloured film-coated tablet.

In one embodiment, the pharmaceutical formulation described herein may be a pink film-coated tablet.

### Pharmaceutical Formulation for Use in a Method of Treatment

In one embodiment there is provided a pharmaceutical formulation which comprises ceralasertib, dibasic calcium phosphate, microcrystalline cellulose, low-substituted hydroxypropyl cellulose and magnesium stearate, as defined herein, for use in a method of treatment of the human or animal body by therapy.

In one embodiment there is provided a pharmaceutical formulation as defined herein for use in a method of producing an ATR inhibitory effect in a warm-blooded animal, such as a man, which comprises administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

In one embodiment there is provided a pharmaceutical formulation as defined herein for use in a method of treating cancer in a warm-blooded animal, such as a man, which comprises administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

In one embodiment there is provided a pharmaceutical formulation as defined herein for use in a method of treating breast cancer, triple negative breast cancer, gastric cancer, chronic lymphocytic leukaemia, head and neck squamous cell carcinoma, non-small cell lung cancer, ovarian cancer, non-Hodgkin's lymphoma, small cell lung cancer, or melanoma, in a warm-blooded animal, such as a man, which comprises administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

In one embodiment there is provided a pharmaceutical formulation as defined herein for use in a method of treating lung cancer, particularly non-small cell lung cancer or small cell lung cancer, in a warm-blooded animal, such as a man, which comprises administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

In one embodiment there is provided a pharmaceutical formulation as defined herein for use in a method of treating melanoma, in a warm-blooded animal, such as a man, which comprises administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

As used herein, the terms "treatment" and "treat" refer to reversing, alleviating, delaying the onset of, or inhibiting the progress of a disease or disorder, or one or more symptoms thereof, as described herein. In some embodiments, treatment may be conducted after one or more symptoms have developed. In other embodiments, treatment may be conducted in the absence of symptoms. For example, treatment may be conducted to a susceptible individual prior to the onset of symptoms (e.g. in light of a history of symptoms and/or in light of genetic or other susceptibility factors). Treatment may also be continued after symptoms have resolved, for example to present or delay their recurrence.

In one embodiment, there is provided a pharmaceutical formulation as defined herein for use in a method of treating cancer in a warm-blooded animal, such as a man, which comprises (1) determining whether or not the warm blooded animal has a cancer receptive to ATR inhibition and (2) if so administering to said animal an effective amount of said pharmaceutical formulation which comprises ceralasertib, dibasic calcium phosphate, microcrystalline cellulose, low-substituted hydroxypropyl cellulose and magnesium stearate, as defined herein.

In one embodiment there is provided a pharmaceutical formulation which comprises ceralasertib, dibasic calcium phosphate, microcrystalline cellulose, low-substituted hydroxypropyl cellulose and magnesium stearate, as defined herein, for use as a medicament.

In one embodiment there is provided a pharmaceutical formulation which comprises ceralasertib, dibasic calcium phosphate, microcrystalline cellulose, low-substituted hydroxypropyl cellulose and magnesium stearate, as defined herein, for use in therapy.

In a not claimed embodiment there is provided the use of a pharmaceutical formulation which comprises ceralasertib, dibasic calcium phosphate, microcrystalline cellulose, low-substituted hydroxypropyl cellulose and magnesium stearate, as defined herein, in the manufacture of a medicament for the production of an ATR inhibitory effect in a warm-blooded animal such as a man.

In a not claimed embodiment there is provided the use of a pharmaceutical formulation which comprises ceralasertib, dibasic calcium phosphate, microcrystalline cellulose, low-substituted hydroxypropyl cellulose and magnesium stearate, as defined herein, in the manufacture of a medicament for the treatment of cancer in a warm-blooded animal such as a man.

In a not claimed embodiment, there is provided the use of a pharmaceutical formulation which comprises ceralasertib, dibasic calcium phosphate, microcrystalline cellulose, low-substituted hydroxypropyl cellulose and magnesium stearate, as defined herein, in the manufacture of a medicament for the treatment of breast cancer, triple negative breast cancer, gastric cancer, chronic lymphocytic leukaemia, head and neck squamous cell carcinoma, non-small cell lung cancer, ovarian cancer, non-Hodgkin's lymphoma, small cell lung cancer, or melanoma.

In a not claimed embodiment, there is provided the use of a pharmaceutical formulation which comprises ceralasertib, dibasic calcium phosphate, microcrystalline cellulose, low-substituted hydroxypropyl cellulose and magnesium stearate, as defined herein, in the manufacture of a medicament for the treatment of melanoma.

In a not claimed embodiment, there is provided the use of a pharmaceutical formulation which comprises ceralasertib, dibasic calcium phosphate, microcrystalline cellulose, low-substituted hydroxypropyl cellulose and magnesium stearate, as defined herein, in the manufacture of a medicament for the treatment of lung cancer, particularly non-small cell lung cancer or small cell lung cancer.

In one embodiment there is provided a pharmaceutical formulation which comprises ceralasertib, dibasic calcium phosphate, microcrystalline cellulose, low-substituted hydroxypropyl cellulose and magnesium stearate, as defined herein, for use in the production of an ATR inhibitory effect in a warm-blooded animal such as a man.

In one embodiment there is provided a pharmaceutical formulation which comprises ceralasertib, dibasic calcium phosphate, microcrystalline cellulose, low-substituted hydroxypropyl cellulose and magnesium stearate, as defined herein, for use in the treatment of cancer in a warm-blooded animal such as a man.

In one embodiment, there is provided a pharmaceutical formulation which comprises ceralasertib, dibasic calcium phosphate, microcrystalline cellulose, low-substituted hydroxypropyl cellulose and magnesium stearate, as defined herein, for use in the treatment of breast cancer, triple negative breast cancer, gastric cancer, chronic lymphocytic leukaemia, head and neck squamous cell carcinoma, non-small cell lung cancer, ovarian cancer, non-Hodgkin's lymphoma, small cell lung cancer, or melanoma.

In one embodiment, there is provided a pharmaceutical formulation which comprises ceralasertib, dibasic calcium phosphate, microcrystalline cellulose, low-substituted hydroxypropyl cellulose and magnesium stearate, as defined herein, for use in the treatment of melanoma.

In one embodiment, there is provided a pharmaceutical formulation which comprises ceralasertib, dibasic calcium phosphate, microcrystalline cellulose, low-substituted hydroxypropyl cellulose and magnesium stearate, as defined herein, for use in the treatment of lung cancer, particularly non-small cell lung cancer or small cell lung cancer.

### DETAILED DESCRIPTION OF THE FIGURES

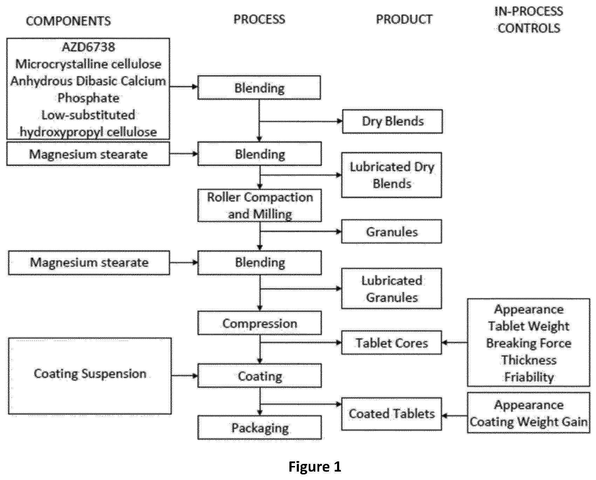

Figure 1 is a process flow diagram depicting the manufacture of a ceralasertib tablet of the present specification.

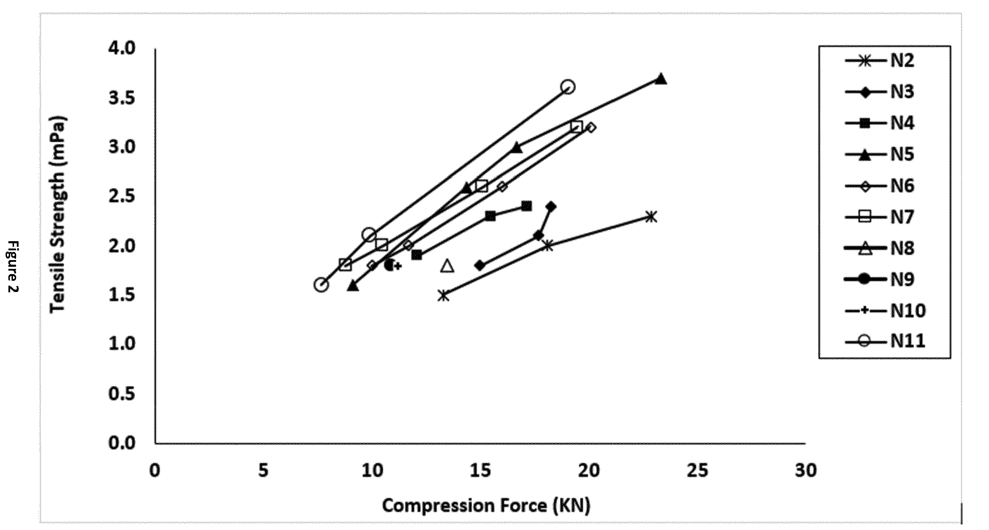

Figure 2 shows the compression profile for a formulation of the present specification (N11) compared to other formulations of ceralasertib (N2-N10) (not according to the invention) and the results from Experiment 1. The results show that N11 requires a lower compression force to achieve the desired tablet tensile strength thus minimising the risk of over-compression issues.

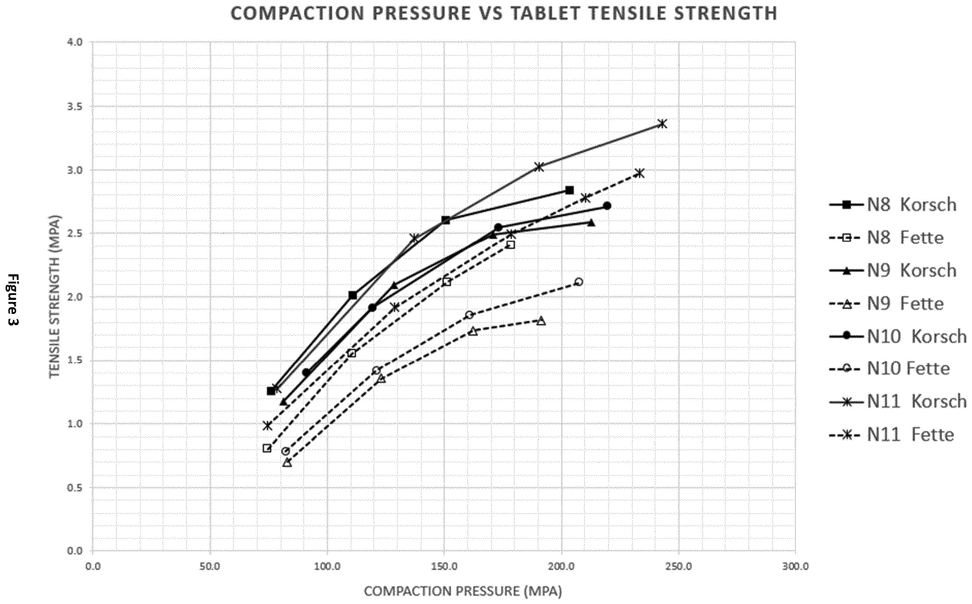

Figure 3 shows compaction pressure vs tensile strength data for formulation variants N8, N9, N10 and N11 (formulation variants N8, N9 and N10 are not according to the invention) from Experiment 2. Comparative formulations N9 and N10 cannot achieve the target 2MPa tensile strength for the tablet at a compression pressure 150 MPa with higher manufacturing speed (such as Fette, 72,000 tablets/hour), which means that the tablets cannot achieve the target mechanical strength at normal compression pressure 100-200 MPa. A formulation of the present specification (N11) can achieve the target tensile strength of >2 MPa and ensure robust mechanical strength for both simulation profiles at a compression pressure 150 MPa.

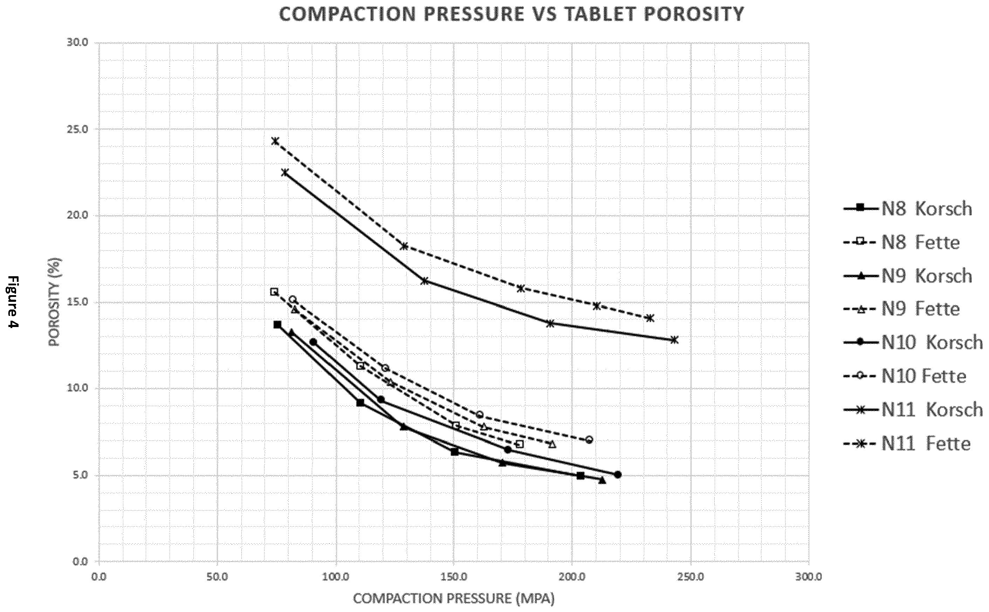

Figure 4 shows compaction pressure vs tablet porosity for formulation variants N8, N9, N10 and N11 (formulation variants N8, N9 and N10 are not according to the invention) from Experiment 2. Tablet porosity is low (<10%) at normal compaction pressures (100-200 MPa) for comparative formulations N8, N9 and N10, which bring the risks of variable dissolution. A formulation of the present specification (N11) has a porosity higher than 10% which minimises the risk of variable dissolution and over-compression.

### EXAMPLES

The excipients used in the Example and Results sections were of the following grades:

**Table 4: Excipients, abbreviations and grades**

| **Excipient name** | **Abbreviation** | **Grade** |
|---|---|---|
| Mannitol | - | Pearlitol-200SD |
| Microcrystalline cellulose | MCC | Avicel PH-102 |
| Dibasic Calcium Phosphate | DCPA | Emcompress^{®} Anhydrous |
| Sodium starch glycolate | SSG | Type A |
| Low-substituted hydroxypropyl | LHPC | LH-31 |
| cellulose | | |
| Hydroxypropyl cellulose | HPC | Klucel EXF |
| Magnesium stearate | - | M F-2-V |

### Example 1

### Preparation of ceralasertib Form A

Form A material was produced by the synthesis route disclosed in WO2011154737, as the Example 2.02 in the route bridging pages 66 and 67. The final product, 4-{4-[(3R)-3-methylmorpholin-4-yl]-6-[1-((R)-S-methylsulfonimidoyl)cyclopropyl]pyrimidin-2-yl}-1H-pyrrolo[2,3-b]pyridine (ceralasertib) Form A, was analysed by XRPD and DSC and found to be crystalline with an onset of melting at about 221.8°C and a peak at about 222.3°C.

### Example 2

### Preparation of ceralasertib Form B

Form B was produced by slurrying Form A in a mixture of 30% isopropyl alcohol and 70% water before placing in a domestic freezer (at a temperature of about -18°C) for about 1 month. After 1 month of storage in a domestic freezer, the sample was removed and allowed to equilibrate at ambient temperature, the cap was then taken off and the slurry left to dry under ambient conditions. The resultant material was determined to be crystalline by XRPD and was also analysed by DSC and had an onset of melting at 53.0°C and a peak at 54.2°C. Alternatively, Form B may be produced by slurrying Form A in a mixture of 30% isopropyl alcohol and 70% water for 2 weeks at a temperature of about 5°C.

### Example 3

### X-Ray Powder Diffraction

Analytical Instrument: Bruker D4. The X-ray powder diffractogram was determined by mounting a sample of the crystalline material on a Bruker single silicon crystal (SSC) wafer mount and spreading out the sample into a thin layer with the aid of a microscope slide. The sample was spun at 30 revolutions per minute (to improve counting statistics) and irradiated with X-rays generated by a copper long-fine focus tube operated at 40kV and 40mA with a wavelength of 1.5418 angstroms. The collimated X-ray source was passed through an automatic variable divergence slit set at V20 and the reflected radiation directed through a 5.89mm anti scatter slit and a 9.55mm detector slit. The sample was exposed for 0.03 seconds per 0.00570° 2-theta increment (continuous scan mode) over the range 2 degrees to 40 degrees 2-theta in theta-theta mode. The instrument was equipped with a position sensitive detector (Lynxeye). Control and data capture was by means of a Dell Optiplex 755 XP professional Workstation operating with Diffrac+ software. Persons skilled in the art of X-ray powder diffraction will understand that the relative intensity of peaks can be affected by, for example, grains above 30 microns in size and non-unitary aspect ratios that may affect analysis of samples. The skilled person will also understand that the position of reflections can be affected by the precise height at which the sample sits in the diffractometer and the zero calibration of the diffractometer. The surface planarity of the sample may also have a small effect. Hence the diffraction pattern data described herein are not to be taken as absolute values.

### Example 4

### Differential Scanning Calorimetry

Analytical Instrument: TA Instruments Q2000 DSC. An amount, typically less than 5mg of material, contained in a standard aluminium pan fitted with a lid was heated over the temperature range 25°C to 300°C at a constant heating rate of 10°C per minute. A purge gas using nitrogen was used - flow rate 50ml per minute.

### Example 5

### Procedure for manufacture of ceralasertib tablets

Ceralasertib tablets were manufactured using conventional blending, dry granulation, and compression processes, according to Good Manufacturing Practice. Routine in-process checks were carried out throughout the manufacturing process.

A summary of a suitable roller compaction process is outlined in Figure 1 and described below:
1. The following ingredients were added to a suitable diffusion mixer: ceralasertib, microcrystalline cellulose, anhydrous dibasic calcium phosphate, low-substituted hydroxypropyl cellulose. The dry ingredients were then mixed together. If needed, materials were pre-screened through an appropriate screen.
2. Intra-granular magnesium stearate was added to the powders, and mixed prior to roller compaction. If needed, material was pre-screened through an appropriate screen.
3. Ribbons were produced by roller compacting the lubricated blend. Subsequently the ribbons were milled into granules by passing the ribbons through a suitable mill.
4. The granules were mixed with extra-granular lubricant (magnesium stearate) using a suitable mixer. If needed, material was pre-screened through an appropriate screen.
5. The lubricated granules were compressed into tablet cores using a suitable tablet press.

The following in-process tests were performed during compression: appearance, tablet weight, breaking force, thickness and friability.

### Example 6

### Preparation of comparative formulations (N2-N10) and a formulation of the present specification (N11)

Formulations N2-N11 were prepared according (or analogous) to the procedure of Example 5. The values quoted in the table are %w/w.

**Table 5: Comparative formulations (N2-N10) and a formulation of the present specification (N11)**

| | **N2** | **N3** | **N4** | **N5** | **N6** | **N7** | **N8** | **N9** | **N10** | **N11** |
|---|---|---|---|---|---|---|---|---|---|---|
| **ceralasertib** | 40 | 40 | 40 | 40 | 40 | 40 | 40 | 40 | 40 | 40 |
| **Mannitol** | 40 | 0 | 0 | 0 | 10 | 0 | 0 | 0 | 0 | 0 |
| **DCPA** | 0 | 37 | 26.5 | 16.5 | 0 | 0 | 0 | 0 | 0 | 15 |
| **MCC** | 13 | 16.5 | 27 | 37 | 44.5 | 48.5 | 48.5 | 53.5 | 58.5 | 33.5 |
| **LHPC** | 0 | 0 | 0 | 0 | 0 | 10 | 10 | 5 | 0 | 10 |
| **SSG** | 5 | 5 | 5 | 5 | 4 | 0 | 0 | 0 | 0 | 0 |
| **Mag Stearate (intra)** | 1 | 1 | 1 | 0.5 | 0.5 | 0.5 | 0.5 | 0.5 | 0.5 | 0.5 |
| **Mag Stearate (extra)** | 1 | 0.5 | 0.5 | 1 | 1 | 1 | 1 | 1 | 1 | 1 |

### RESULTS

### Experiment 1

### Compression Profile

The compression profile depicts the tablet tensile strength as a function of the applied compaction force for all batches. This profile is normalised for the punch tip face area and tablet geometry, allowing for a true comparison of formulation robustness independent of tablet size.

All batches were compressed separately using a suitable rotary tablet press (e.g. Korsch XL100) with the same punch design under different compression forces (N8, N9 and N10 were compressed with one force only). Breaking force and thickness of the produced tablets were characterized using suitable hardness and thickness measurement equipment. Tensile strength was calculated in terms of breaking force and dimension of produce tablets. The results are presented in Figure 2. Formulation variants N2-N10 are not according to the invention.

### Experiment 2

### Compaction pressure vs tensile strength and table porosity for N8-N11

The compaction profile for N8-N11 was measured using a suitable compaction simulator which can mimic development vs commercial tableting and compacting processes. Formulation variants N8, N9 and N10 are not according to the invention. Round, flat-faced tablets were compressed with single punch, and in-die data were evaluated based on different simulation profiles. Korsch simulates development level production (12,000 tablets/hour) and Fette simulates commercial level production at a higher manufacturing speed (72,000 tablets/hour). Compaction properties and the effects of varying compression force and speed on tablet properties were investigated. The results are presented in Figure 3 and Figure 4.

## Claims

1. A pharmaceutical formulation which comprises ceralasertib, dibasic calcium phosphate, microcrystalline cellulose, low-substituted hydroxypropyl cellulose and magnesium stearate.

2. A pharmaceutical formulation as claimed in claim 1 which comprises 30-50 % w/w ceralasertib.

3. A pharmaceutical formulation as claimed in any one of the preceding claims wherein the ceralasertib is ceralasertib Form A with an X-ray powder diffraction pattern with at least two specific peaks at about 2-theta = 21.2° and 17.2° as measured using CuKα radiation.

4. A pharmaceutical formulation as claimed in any one of the preceding claims which comprises 10-16% w/w anhydrous dibasic calcium phosphate.

5. A pharmaceutical formulation as claimed in any one of the preceding claims which comprises 30-40% w/w microcrystalline cellulose.

6. A pharmaceutical formulation as claimed in any one of the preceding claims which comprises 5-20% w/w low substituted hydroxypropyl cellulose.

7. A pharmaceutical formulation as claimed in any one of the preceding claims which comprises 0.5-2.5 % w/w magnesium stearate.

8. A pharmaceutical formulation as claimed in any one of the preceding claims which comprises 160mg of ceralasertib.

9. A pharmaceutical formulation as claimed in any one of claims 1 to 7 which comprises 120mg of ceralasertib.

10. A pharmaceutical formulation as claimed in any one of claims 1 to 7 which comprises 80mg of ceralasertib.

11. A pharmaceutical formulation as claimed in any one of the preceding claims which comprises:
40% w/w ceralasertib;
15% w/w anhydrous dibasic calcium phosphate;
33.5% w/w microcrystalline cellulose;
10% w/w low substituted hydroxypropyl cellulose; and
1.5% w/w magnesium stearate.

12. A pharmaceutical formulation as claimed in any one of the preceding claims that is a tablet formulation.

13. A pharmaceutical formulation as claimed in claim 12 additionally comprising a coloured film coating.

14. A pharmaceutical formulation as claimed in any one of the preceding claims for use as a medicament.

15. A pharmaceutical formulation as claimed in any one of claims 1-13 for use in the treatment of breast cancer, triple negative breast cancer, gastric cancer, chronic lymphocytic leukaemia, head and neck squamous cell carcinoma, non-small cell lung cancer, ovarian cancer, non-Hodgkin's lymphoma, small cell lung cancer, or melanoma.

## Patentansprüche

1. Pharmazeutische Formulierung, die Ceralasertib, zweibasisches Calciumphosphat, mikrokristalline Cellulose, niedrig substituierte Hydroxypropylcellulose und Magnesiumstearat umfasst.

2. Pharmazeutische Formulierung nach Anspruch 1, die zu 30-50 Gew.-% Ceralasertib umfasst.

3. Pharmazeutische Formulierung nach einem der vorstehenden Ansprüche, wobei es sich bei dem Ceralasertib um Ceralasertib Form A mit einem Röntgenpulverbeugungsmuster mit mindestens zwei spezifischen Spitzenwerten bei etwa 2-Theta = 21,2° und 17,2° handelt, gemessen unter Verwendung von CuKα-Strahlung.

4. Pharmazeutische Formulierung nach einem der vorstehenden Ansprüche, die zu 10-16 Gew.-% wasserfreies zweibasisches Calciumphosphat umfasst.

5. Pharmazeutische Formulierung nach einem der vorstehenden Ansprüche, die zu 30-40 Gew.-% mikrokristalline Cellulose umfasst.

6. Pharmazeutische Formulierung nach einem der vorstehenden Ansprüche, die zu 5-20 Gew.-% niedrig substituierte Hydroxypropylcellulose umfasst.

7. Pharmazeutische Formulierung nach einem der vorstehenden Ansprüche, die zu 0,5-2,5 Gew.-% Magnesiumstearat umfasst.

8. Pharmazeutische Formulierung nach einem der vorstehenden Ansprüche, die 160 mg Ceralasertib umfasst.

9. Pharmazeutische Formulierung nach einem der Ansprüche 1 bis 7, die 120 mg Ceralasertib umfasst.

10. Pharmazeutische Formulierung nach einem der Ansprüche 1 bis 7, die 80 mg Ceralasertib umfasst.

11. Pharmazeutische Formulierung nach einem der vorstehenden Ansprüche, die umfasst:
zu 40 Gew.-% Ceralasertib;
zu 15 Gew.-% wasserfreies zweibasisches Calciumphosphat;
zu 33,5 Gew.-% mikrokristalline Cellulose;
zu 10 Gew.-% niedrig substituierte Hydroxypropylcellulose; und
zu 1,5 Gew.-% Magnesiumstearat.

12. Pharmazeutische Formulierung nach einem der vorstehenden Ansprüche, die eine Tablettenformulierung ist.

13. Pharmazeutische Formulierung nach Anspruch 12, die zusätzlich einen farbigen Filmüberzug umfasst.

14. Pharmazeutische Formulierung nach einem der vorstehenden Ansprüche zur Verwendung als ein Arzneimittel.

15. Pharmazeutische Formulierung nach einem der Ansprüche 1 bis 13 zur Verwendung bei der Behandlung von Brustkrebs, dreifach negativem Brustkrebs, Magenkrebs, chronischer lymphatischer Leukämie, Plattenepithelkarzinom in Kopf und Hals, nicht kleinzelligem Lungenkrebs, Eierstockkrebs, Non-Hodgkin-Lymphom, kleinzelligem Lungenkrebs oder Melanom.

## Revendications

1. Formulation pharmaceutique qui comprend du céralasertib, du phosphate de calcium dibasique, de la cellulose microcristalline, de l'hydroxypropylcellulose faiblement substituée et du stéarate de magnésium.

2. Formulation pharmaceutique selon la revendication 1, qui comprend 30 à 50 % p/p de céralasertib.

3. Formulation pharmaceutique selon l'une quelconque des revendications précédentes, dans laquelle le céralasertib est la forme A de céralasertib avec un schéma de diffraction des rayons X sur poudre présentant au moins deux pics spécifiques à environ 2-theta = 21,2° et 17,2°, mesurés à l'aide de la radiation CuKα.

4. Formulation pharmaceutique selon l'une quelconque des revendications précédentes, qui comprend 10 à 16 % p/p de phosphate de calcium dibasique anhydre.

5. Formulation pharmaceutique selon l'une quelconque des revendications précédentes, qui comprend 30 à 40 % p/p de cellulose microcristalline.

6. Formulation pharmaceutique selon l'une quelconque des revendications précédentes, qui comprend 5 à 20 % p/p d'hydroxypropylcellulose faiblement substituée.

7. Formulation pharmaceutique selon l'une quelconque des revendications précédentes, qui comprend 0,5 à 2,5 % p/p de stéarate de magnésium.

8. Formulation pharmaceutique selon l'une quelconque des revendications précédentes, qui comprend 160 mg de céralasertib.

9. Formulation pharmaceutique selon l'une quelconque des revendications 1 à 7, qui comprend 120 mg de céralasertib.

10. Formulation pharmaceutique selon l'une quelconque des revendications 1 à 7, qui comprend 80 mg de céralasertib.

11. Formulation pharmaceutique selon l'une quelconque des revendications précédentes, qui comprend :
40 % p/p de ceralasertib ;
15 % p/p de phosphate de calcium dibasique anhydre ;
33,5 % p/p de cellulose microcristalline ;
10 % p/p d'hydroxypropylcellulose faiblement substituée ; et
1,5 % p/p de stéarate de magnésium.

12. Formulation pharmaceutique selon l'une quelconque des revendications précédentes qui est sous forme de comprimé.

13. Formulation pharmaceutique selon la revendication 12, comprenant en outre un revêtement de film coloré.

14. Formulation pharmaceutique selon l'une quelconque des revendications précédentes pour une utilisation en tant que médicament.

15. Formulation pharmaceutique selon l'une quelconque des revendications 1 à 13, destinée à être utilisée dans le traitement du cancer du sein, du cancer du sein triple négatif, du cancer gastrique, de la leucémie lymphocytaire chronique, du carcinome épidermoïde de la tête et du cou, du cancer du poumon non à petites cellules, du cancer de l'ovaire, du lymphome non hodgkinien, du cancer du poumon à petites cellules ou du mélanome.
